# EUROPÄISCHE PATENTANMELDUNG

(11) **EP 1 488 855 A1**
(43) Veröffentlichungstag der Anmeldung: **22.12.2004**
(21) Anmeldenummer: 04450123.7
(22) Anmeldetag: 07.06.2004
(51) Int. Cl.: B03B 9/06, B03B 5/32, C02F 11/04, C02F 11/12, C12M 1/107, B30B 9/30, C12P 5/02

(54) **Verfahren und Anlage zur Herstellung von Biogas aus Biomüll**

(30) Priorität: 18.06.2003 AT 9522003
(71) Anmelder: Elektrotechnik Reiter, 3454 Reidling (AT)
(72) Erfinder: Reiter, Arno, 3454 Reidling (AT)
(74) Vertreter: Schwarz, Albin, Dr.

(57) **Zusammenfassung**

Bei einem Verfahren zur Herstellung von Biogas aus Biomüll, wobei der Biomüll zerkleinert und biologisch zumindest teilweise abgebaut wird, wird der Biomüll nach Zerkleinerung und vor dem zumindest teilweisen biologischen Abbau unter Abgabe von Wasser verpresst.

## Beschreibung

Die Erfindung betrifft ein Verfahren zur Herstellung von Biogas aus Biomüll, wobei der Biomüll zerkleinert und zumindest teilweise biologisch abgebaut wird. Weiters betrifft die Erfindung eine Anlage zur Herstellung von Biogas aus Biomüll, umfassend einen Sammelbehälter für den Biomüll, eine mit dem Sammelbehälter leitungsmäßig verbundene Zerkleinerungsvorrichtung für den Biomüll und eine Vergärungsvorrichtung.

Die Entsorgung von Biomüll, also Küchen- und Gartenabfällen, wirft in den letzten Jahren immer größere Probleme auf. Küchenabfälle, die in Österreich aufgrund steigenden Wohlstands, Industrie und Fremdenverkehr stetig anwachsen, müssen zu Tausenden Tonnen durch Österreich transportiert werden, um sie der Entsorgung zuzuführen. Die Transportkosten sind hoch und die Transporte belasten zudem die Umwelt, da sie vorwiegend mittels LKW durchgeführt werden.

Die neue Hygieneverordnung schreibt vor, dass Küchenabfälle in Zukunft nicht mehr unbehandelt entsorgt, d.h. deponiert oder verfüttert, werden dürfen. Durch Vergärung mit Methan produzierenden Mikroorganismen kann Biomüll in Biogas und kompostierbares bzw. deponierfähiges Material umgewandelt werden. Die bereits bestehenden Biogasanlagen, die nur einen Bruchteil des anfallenden Biomülls entsorgen, sind jedoch zu klein und von ihrer Anzahl her zu gering, um die zukünftigen Mengen an zu entsorgendem Biomüll verarbeiten zu können.

Die DE 44 23 099 A beschreibt ein Verfahren zur Vergärung von Biomüll, bei dem der Biomüll gemischt, zerkleinert und mit vorgewärmtem flüssigem Vergärungsprodukt versetzt wird, bevor er zur Vergärung in einen Fermenter eingespeist wird. Dieses Verfahren macht jedoch ein eigenes System für den im fließ- bzw. pumpfähigen Zustand befindlichen Biomüll erforderlich.

Gemäß WO 98/41646 A wird pflanzliche Biomasse verwertet, indem sie zerkleinert und in Schneckenpressen verpresst wird, die abgeschiedene feststoffreiche Phase mit Dampf im Überdruck sterilisiert, gekühlt und danach einer Fermentation unterzogen wird. Nachteilig bei diesem Verfahren ist jedoch der hohe technische und apparative Aufwand. Zudem ist die Verarbeitung bestimmter organischer Abfallprodukte, wie z.B. Knochen, Fisch, Fleisch, mit diesem Verfahren nicht oder nur beschränkt möglich.

Die DE 198 05 045 A beschreibt ein Verfahren zur Methanisierung schüttfähiger, stapelbarer oder stückigmachbarer Biomassen, wobei der in einem Behälter angeordneten, von einer luftundurchlässigen Hülle umgebenen Biomasse ein Impfmaterial zugesetzt, und die so gebildete Reaktionsmasse unter Luftabschluss vergoren wird. Als Einsatzmaterial werden zum Beispiel Biomassen in Form von Pressballen verwendet.

Die DE 100 50 425 A betrifft ein Verfahren zur Methanisierung von biogenen Stückgütern in Trockenfermentern, bei dem aus den Stückgütern locker aufgepresste, mit einem Kunststoffnetz umwickelte Ballen hergestellt werden, um eine spätere Infiltration und Perkolation durch das Impfmaterial zu ermöglichen und gleichzeitig die Transportfähigkeit sicherzustellen.

Die DE 100 20 832 A lehrt ein Verfahren zur Energiegewinnung aus saisonal anfallenden organischen Abfällen, wobei die Abfälle verpresst und durch Wasserentzug auf einen Wassergehalt von ca. 10-30 % gebracht werden, um eine Vergärung zu verhindern, damit sie bei Bedarf vor der Nassfermentation gelagert werden können.

Die Erfindung stellt sich die Aufgabe, die oben genannten Probleme und Nachteile zu überwinden und ein Verfahren und eine Anlage bereitzustellen, welche die Herstellung von Biogas aus Biomüll ermöglichen, wobei die Menge an zu entsorgendem Biomüll nach dem Sammeln verringert wird, wodurch Transportkosten und Umweltbelastung gesenkt werden können.

Diese Aufgabe wird bei einem Verfahren der eingangs genannten Art erfindungsgemäß dadurch gelöst, dass der Biomüll nach Zerkleinerung und vor dem zumindest teilweisen biologischen Abbau unter Abgabe von Wasser verpresst wird.

Das zurückbleibende halbfeuchte Material weist ein geringeres Volumen sowie ein geringeres Gewicht auf, so dass insgesamt weniger Entsorgungskosten anfallen.

In einer bevorzugten Ausführungsform wird die Verpressung derart durchgeführt, dass der durch die Abgabe von Wasser bewirkte Gewichtsverlust des Biomülls mindestens 50% beträgt. Vorzugsweise beträgt der Gewichtsverlust des Biomülls mindestens 70%, insbesondere mindestens 75%.

Durch das Verpressen kann die Menge an zu entsorgendem Biomüll somit auf etwa 30% verringert werden. Das entstandene Granulat weist dann eine Trockensubstanz von ca. 25% auf und kann im Gegensatz zu unbehandeltem Biomüll auch ohne Behälter transportiert werden.

In einer weiteren bevorzugten Ausführungsform erfolgt der zumindest teilweise biologische Abbau durch Trockenfermentation. Diese Art der Vergärung bietet wesentliche Vorteile, die im folgenden erläutert werden.

Bei der Trockenfermentation handelt es sich um ein Batch-Vefahren, das im Gegensatz zum Flüssigvergärungsverfahren keine Umsetzung des Biomülls in einen fließ- bzw. pumpfähigen Zustand benötigt.

Bei der einstufigen Prozessführung finden die verschiedene Abbaureaktionen (Hydrolyse, Säure-, Essig- und Methanbildung) in einem Reaktor statt, wohingegen bei der Flüssigvergärung die einzelnen Prozesse räumlich getrennt in einer zwei- oder mehrstufigen Prozessführung ablaufen, woraus ein erheblich höherer apparativer und maschineller Aufwand resultiert.

Aufgrund der wesentlich höheren Trockenmassegehalte liegt der Anteil der Prozessenergie bei der Trockenfermentation im Vergleich zur Flüssigvergärung deutlich niedriger (max. 5% gegenüber bis zu 45% bei der Nassfermentation). Die Trockenfermentation liefert einen Methangehalt von bis zu 80% (pro m³ Ausgangsmaterial) und ermöglicht, bezogen auf die organische Trockenmasse, eine mindestens gleiche Gasausbeute wie die konventionelle Nassvergärung.

Der Schwefelgehalt des erzeugten Biogases, der bei der Verwertung über Gas- und Zündstrahlmotoren zu extremem Verschleiß führt, ist bei der Trockenfermentation besonders niedrig und macht daher eine zusätzliche Gasentschwefelung überflüssig.

Der Biomüll kann nach Abschluss des Fermentationsvorgangs in einer herkömmlichen Nachkompostieranlage zu Kompost verarbeitet werden.

Die erfindungsgemäße Anlage ist dadurch gekennzeichnet, dass zwischen der Zerkleinerungsvorrichtung und der Vergärungsvorrichtung eine Presse vorgesehen ist.

Vorzugsweise ist die Vergärungsvorrichtung eine Trockenfermentationsvorrichtung.

Die Erfindung wird nachfolgend anhand eines Beispiels näher erläutert.

### Beispiel:

Über eine oder mehrere Eingabestationen (beispielsweise ins Spülbecken integriert), die zum Beispiel in der Küche verteilt sein können, wird organischer, entwässerbarer Abfall (Obst-, Gemüse-, Fisch-, Schalentier-, Fleisch-, Knochenabfälle, Muschel-, Nuss- und Eierschalen, etc.) eingebracht. Zusammen mit Leitungswasser fließt dieser Brei über ein Rohr- oder Schlauchsystem in eine Sammelanlage. Es entstehen dabei weder eine Geruchsbelästigung noch andere mit Biomüll verbundene Probleme.

Bei maximalem Füllstand des Sammelbehälters schaltet sich automatisch eine Nachzerkleinerung mit integrierter Pumpe und der zugehörigen Entwässerungsstation ein. Von hier gelangt der Biomüll in eine zentrifugale Presse, wo die Aufspaltung in feste und flüssige Stoffe erfolgt. Der nun entstandene feste organische Abfall (Volumen etwa um 85%, Gewicht um etwa 75% reduziert) wird in einem bereitgestellten Behälter gesammelt.

Durch gezieltes, zeitgesteuertes Zuführen des zerkleinerten Biomülls zur zentrifugalen Presse (z.B. Pause 4-10 sec., Laufzeit 10-30 sec.) kann ein Trockensubstanzgehalt, je nach Material und Zeittaktung, zwischen 15% und 45% erreicht werden. Biomüll mit einem derartigen Trockensubstanzgehalt stellt ein ausgezeichnetes Grundmaterial für eine Trockenfermentation dar.

Die Behälter werden zum Beispiel mittels LKW zur Vergärungsvorrichtung gebracht. Um die ökologische Kette zu schließen, können insbesondere biogasbetriebene Fahrzeuge zum Transport eingesetzt werden.

Die Biomüllentwässerungsanlagen können aber auch direkt mit einer Trockenfermentationsvorrichtung gekoppelt sein. Hierfür werden nur ein zusätzliches Zwischenlager für den anfallenden ausgepressten Biomüll sowie ein Fettabscheider benötigt, der die ausgepresste Flüssigkeit vom Fett trennt, wobei das Fett über einen Wärmetauscher aufgeheizt und als hoch biogenes Material zur Impfung im Fermenter herangezogen werden kann.

### Trockenfermentation

Dem im Behälter (Fermenter) eingelagerten, von einer gasdichten Betonhülle umgebenen Biomüll wird ein Impfmaterial zugesetzt, und die so gebildete Reaktionsmasse wird unter Luftabschluss vergoren. Ein Rühren oder Umpumpen sowie die tägliche Zugabe von Gärmaterial entfällt.

Die Temperierung und Beimpfung des Biomülls erfolgt einerseits über die Rezirkulation des anfallenden Perkolats ("gelöste" Methanbakterien), welches in einem separaten Behälter mit installiertem Wärmetauscher aufgeheizt wird, andererseits wird eine Wärmezufuhr über ein Fußbodenheizsystem sichergestellt. Dadurch kann Biomüll mit einem Trockensubstanzgehalt von über 50% vergoren werden.

Der mit einer Temperatur von bis zu 65°C in den Behälter eingebrachte Biomüll wird nach anfänglicher Abkühlung bei etwa 40°C gehalten. In diesem Klima verweilt das Material etwa 25-35 Tage. Das dabei entstehende Gas wird abgeleitet, mittels eines Gasverdichters komprimiert und in einen Druckspeicher überführt. Das abgezogene Gas-Luft-Gemisch wird über einen Biofilter gereinigt und in die Atmosphäre abgelassen. Das gewonnene Biogas wird wie bei der Nassvergärung durch Verbrennung in einem Blockheizkraftwerk zur Strom- und Wärmegewinnung eingesetzt.

Sind der Gär- und der anschließende Trocknungsprozess abgeschlossen, werden die Container entleert. Durch ein Sicherheitssystem aus Luft- und Methansensoren wird verhindert, dass die Behälter vor der Absaugung des restlichen Methans geöffnet werden. Der Biomüll gelangt ohne weitere Bearbeitung und Zusätze in einen nachgeschalteten Kompostiervorgang, wo er mit moderner Technik zu hochwertigem Kompost verarbeitet wird, der in Gartenbaubetrieben, in der Landwirtschaft und von privaten Endverbrauchern als wertvolle Alternative zu herkömmlichen Produkten eingesetzt werden kann. Auf diese Weise entstehen in den Arbeitsgängen keine Abfallprodukte, die entsorgt werden müssten, sondern verwertbare Endprodukte (Strom, Wärme, Kompost, Dünger), die auch zum Teil in der Anlage selbst eingesetzt werden können.

Im Vergleich zur Nassvergärung ist bei der Trockenfermentation die Materialbearbeitung in der Vorbereitung nicht so aufwendig. Der zu vergärende Biomüll muss nicht mehr in einen pump- bzw. fließfähigen Zustand gebracht werden. Auch die sich im Biomüll befindenden Störstoffe, die mit teuren Sortiermaschinen aussortiert werden müssten, können mittels des erfindungsgemäßen Verfahrens in die Vergärung eingebracht werden, ohne dass Geräte oder Maschinen verstopfen.

Bis auf die Befüllung und Entnahme des Biomülls durch Radlader oder andere Füllwerkzeuge kann die komplette Anlage vollautomatisch gesteuert und geregelt werden. Störungen können dabei sofort erkannt und über Signale gemeldet werden.

Die erfindungsgemäße Anlage zeichnet sich dadurch aus, dass qualitativ hochwertiges Biogas (mit hohen Methangehalten und geringem Schwefelanteil) gewonnen wird. Die Gasausbeute ist bezogen auf die eingelagerte organische Trockenmasse vergleichbar mit der konventionellen Flüssigfermentation. Zusätzlich kann die Kapazität einer derartigen Anlage aufgrund ihrer "Modulbauweise" leicht erweitert werden. Für die Trockenfermentationsvorrichtung sind außerdem die Baukosten vergleichsweise gering.

## Patentansprüche

1. Verfahren zur Herstellung von Biogas aus Biomüll, wobei der Biomüll zerkleinert und biologisch zumindest teilweise abgebaut wird, **dadurch gekennzeichnet, dass** der Biomüll nach Zerkleinerung und vor dem zumindest teilweisen biologischen Abbau unter Abgabe von Wasser verpresst wird.

2. Verfahren nach Anspruch 1, **dadurch gekennzeichnet, dass** die Verpressung derart durchgeführt wird, dass der durch die Abgabe von Wasser bewirkte Gewichtsverlust des Biomülls mindestens 50% beträgt.

3. Verfahren nach Anspruch 2, **dadurch gekennzeichnet, dass** der Gewichtsverlust des Biomülls mindestens 70%, vorzugsweise mindestens 75%, beträgt.

4. Verfahren nach einem der Ansprüche 1 bis 3, **dadurch gekennzeichnet, dass** der zumindest teilweise biologische Abbau durch Trockenfermentation erfolgt.

5. Anlage zur Herstellung von Biogas aus Biomüll, umfassend einen Sammelbehälter für den Biomüll, eine mit dem Sammelbehälter leitungsmäßig verbundene Zerkleinerungsvorrichtung für den Biomüll und eine Vergärungsvorrichtung, **dadurch gekennzeichnet, dass** zwischen der Zerkleinerungsvorrichtung und der Vergärungsvorrichtung eine Presse vorgesehen ist.

6. Anlage nach Anspruch 5, **dadurch gekennzeichnet, dass** die Vergärungsvorrichtung eine Trockenfermentationsvorrichtung ist.
